Europäisches Patentamt

European Patent Office

Office européen des brevets

(11) Publication number: **0 102 458**
**B1**

## (12) EUROPEAN PATENT SPECIFICATION

(45) Date of publication of patent specification: **04.06.86**

(21) Application number: **83105533.0**

(22) Date of filing: **06.06.83**

(51) Int. Cl.⁴: **A 61 M 1/14**

(54) Device for measuring of concentration of a low molecular weight compound in a complex medium.

(30) Priority: **15.06.82 SE 8203695**

(43) Date of publication of application:
**14.03.84 Bulletin 84/11**

(45) Publication of the grant of the patent:
**04.06.86 Bulletin 86/23**

(84) Designated Contracting States:
**AT BE CH DE FR GB IT LI LU NL**

(56) References cited:
**EP-A-0 004 760**
**DE-A-2 658 101**
**US-A-3 512 517**
**US-A-3 884 640**

(73) Proprietor: **Gambro Lundia AB**
**Box 10101**
**S-220 10 Lund (SE)**

(72) Inventor: **Claren, Jan Seivert**
**Protokollgränden 38**
**S-222 47 Lund (SE)**
Inventor: **Olsson, Lars-Göran**
**Docentgatan 6**
**S-223 63 Lund (SE)**
Inventor: **Hallberg, Birger Ragnar**
**Kamrersgatan 5**
**S-237 00 Bjärred (SE)**

(74) Representative: **Boberg, Nils Gunnar Erik et al**
**Gambro AB Patent Department Box 10101**
**S-220 10 Lund (SE)**

Courier Press, Leamington Spa, England.

## Description

Technical Field

This invention relates to a device for measuring of the concentration of a low molecular weight compound in a complex medium, comprising a dialyzer, an enzyme reactor and a measuring unit in communication with each other through a channel system.

More specifically, this invention relates to a device for measuring of the concentration of glucose in blood of a patient.

Technical Background

A device of the disclosed kind, specifically for measuring of the concentration of glucose in blood, is known through inter alia US—A— 4 229 542, wherein the blood is passed through the dialyzer to form a glucose-containing dialysate which is thereafter passed through the enzyme reactor in which said glucose is converted to a more easily measurable compound, the concentration of which is measured in the following measuring unit as an indirect measure of the concentration of glucose in the blood.

Similar devices are also known through GB—A—1 581 354 and US—A—4 266 021. GB—A—1 564 788 describes a similar device without a dialyzer for measuring of concentration of glucose in blood.

A major and serious problem with these known arrangements is that they use a great number of separate tubes as a channel system for joining the involved components. This problem affects not only the manufacturing and mounting, but also the transport and handling. The tubes are very small and require accurate dimensions in order to obtain thorough measurings. Unfortunately, it is very difficult to standardize the manufacturing in such a degree that this requirement is always achieved. Since several tubes have mutually different dimensions, and this difference per se is very small, the risk will be big that an improper tube dimension is used in a wrong place in the mounting. For example, it can be very easy to use a tube being adapted to lead blood in a place which is adapted to be coupled to a tube for dialysis solution and vice versa. The ready-for-use tube set is supplied and stored in a sterilized condition, but since the tubes due to their small dimensions are extremely sensitive to external influences in for example incautious handling during transport the risk is big that these can be damaged with the consequence that the sterility is lost. A further serious drawback due to the fact that the tubes are small and flexible is that they during use are very difficult to fix in a determined pattern, such that determined flow pathways for the liquids are achieved, whereby disturbances in the measuring accuracy often will follow. Disturbances in the measuring results can also be caused by the fact that blood has a tendency to coagulate when disturbed in its flow pathway, especially at couplings, whereby the risk for coagulating increases when the number of used couplings is increased.

An object of the invention is therefore to provide an improved device which is more easy to manufacture and install and which makes it possible with a larger degree of reproducible measuring accuracy than before to determine the concentration of a low molecular compound in a complex medium.

This object is achieved by means of the device as defined in the accompanying claims and which will be described in the following.

Brief Description of the Invention

According to the invention there is provided a device for measuring of the concentration of a low molecular weight compound in a complex medium, which comprises a dialyzer, an enzyme reactor, and a measuring unit in communication with each other through a channel system. The device is characterized in that said channel system is composed of grooves which are arranged on one side of a base plate and which are covered by one or more plates being piled over each other.

The base plate is preferably manufactured of a rigid material with said grooves being provided in connection with for example the molding of said plate. Thereby it is possible to assure a great accuracy in the pattern of grooves from one plate to another even in large scale production. Furthermore, it is also possible to automize the manufacturing, which, on the contrary, was not satisfactorily possible for the corresponding tubes in the known devices.

The grooves on the base plate are conveniently covered by a corresponding plate, preferably of a soft material, whereby a good tightening between the formed channels is achieved when a top plate, being piled upon the soft plate, of rigid material is adapted under a proper pressure to securely clamp the soft plate between the base plate and the top plate. This clamping can be provided for example by means of a rivet joint, screw joint, or a welding joint between the base plate and the top plate.

Preferably the base plate is manufactured of a transparent material, whereby it is possible to visually monitor flows of coloured liquids when the device subsequently is being used.

The dialyzer, enzyme reactor and measuring unit are preferably arranged on the upper side of the top plate of rigid material and in connection with respective grooves in the base plate via penetrating holes in the soft plate and the top plate.

Furthermore, in order to assure a good tightening in the formed channels through the soft intermediate plate, this latter is preferably provided with perforated taps which with close fitting extend into the respective hole in the rigid top plate.

Preferably, the device comprises a temperature sensor to measure the temperature of the dialysate which flows out of the dialyzer. Conveniently, said temperature sensor is provided between the top plate and the intermediate plate in contact with said dialysate, whereby good isolation of possible electrical lines in the temperature sensor is provided.

Furthermore, the channel system may be interrupted by a pump segment or tubes being arranged over a pump hole through the plates, whereby a simple means for pumping the respective liquids in the channel system is created.

Furthermore, the device preferably comprises also a deaerator consisting of a hole through the intermediate plate and the top plate, which is covered by a hydrophobic membrane and which is in communication with the dialysate from the dialyzer.

Since the enzyme reactor is a so-called piece of perishable and therefore cannot be stored for a long time, the top plate (upon which the enzyme reactor is adapted to be arranged) preferably is provided with means such that the enzyme reactor can be readily mounted upon this immediately before use.

The dialyzer may be a hollow fiber, a plate, or a tube dialyzer, but is preferably a plate dialyzer and built up in essentially the same way as the channel system, i.e. of a rigid base plate and a rigid top plate having therebetween a soft intermediate plate provided with a channel over which a semipermeable membrane is arranged in order thereby to provide tight flow pathways on each side of the membrane for for example the complex medium and the dialysis solution, respectively. Preferably, the rigid base plate is partly composed of the rigid top plate which forms the channel system and upon which the dialyzer is adapted to be arranged.

Brief Description of Drawings

The invention will be described in more detail in the following with reference to the enclosed drawings, wherein

Fig. 1 shows a preferred embodiment of the device according to the invention,

Fig. 2 is a bottom view of the device in Fig. 1,

Figs. 3—5 show preferred embodiments of the respective plates forming the channel system in the device of Figs. 1 and 2, and wherein

Fig. 6 shows a preferred embodiment of one plate of the dialyzer.

Detailed Description of Drawings

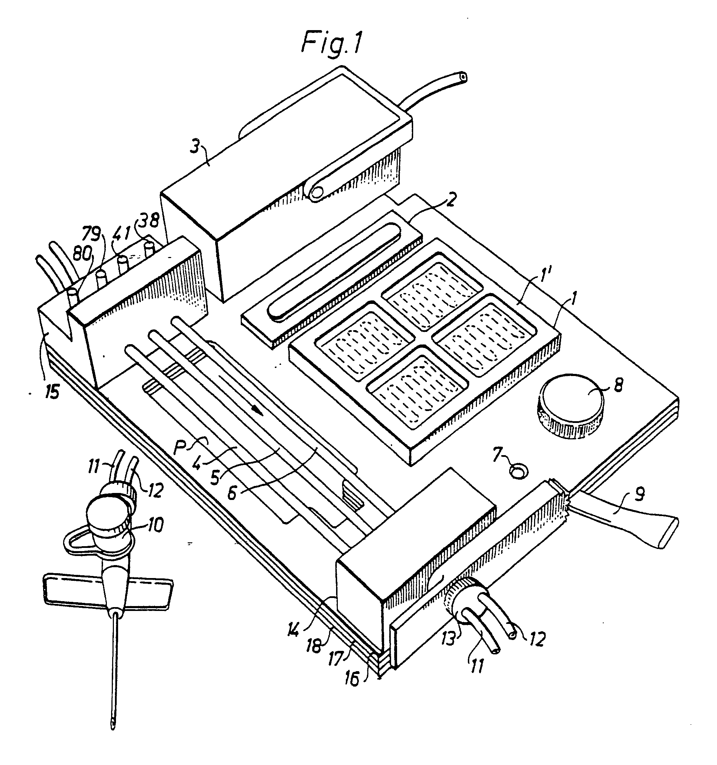

As shown in Fig. 1 the device according to the invention comprises a dialyzer 1, an enzyme reactor 2, and a measuring unit 3, which are in communication with each other through a channel system which is only partly shown in Fig. 1 in the form of tubes 4, 5, 6. Furthermore, the device comprises a deaerator 7, a flow changing means 8 and a temperature sensor 9. Finally, the device comprises a tube set comprising a cannula 10, tubes 11, 12 and a changing valve 13, which is connected to a first connecting piece 14 for communication with the channel system, as will be described. A second connecting piece 15 is provided opposite to the first one, whereby the tubes 4, 5, 6 are arranged between these over a hole P through a stack of plates 16, 17, 18 to be actuated by a not shown pumping device in the direction of the arrow.

The above described elements, i.e. enzyme reactor, measuring unit and flow changing means, are per se known in a device of the disclosed kind and will therefore not be described herein in more detail. For further information reference is instead made to one or more of the above-mentioned patents.

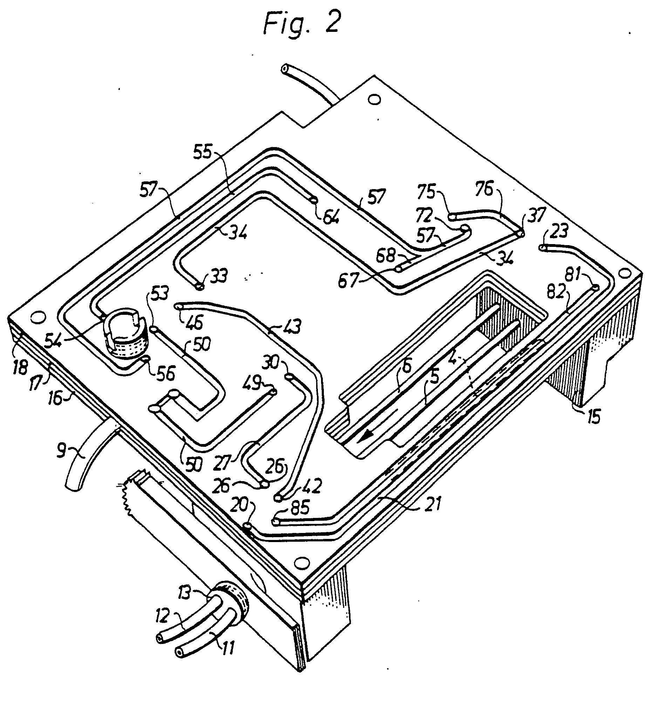

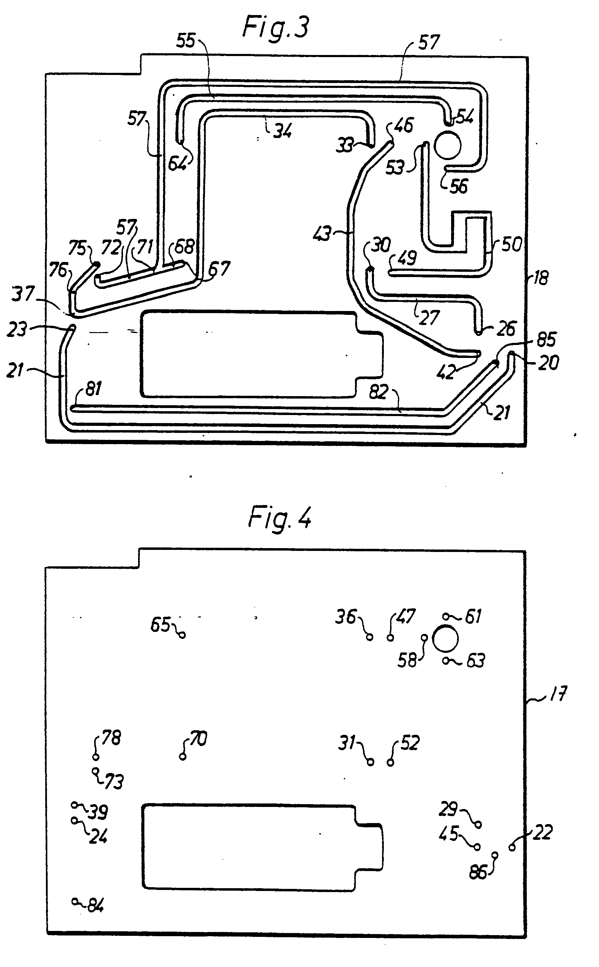

The plates shown in Figs. 3—5 are adapted to be stacked one over the other with the plate 18 at the bottom and thereafter in turn plate 17 and plate 16 at the top to form the stack shown in Figs. 1 and 2. Preferably, the base plate 18 and the top plate 16 are manufactured of rigid material, while the intermediate plate 17 is manufactured of a soft material, whereby good tightening between the formed channels is achieved when the top plate and the base plate are firmly clamped against each other by for example a welding joint, screw joint, or rivet joint.

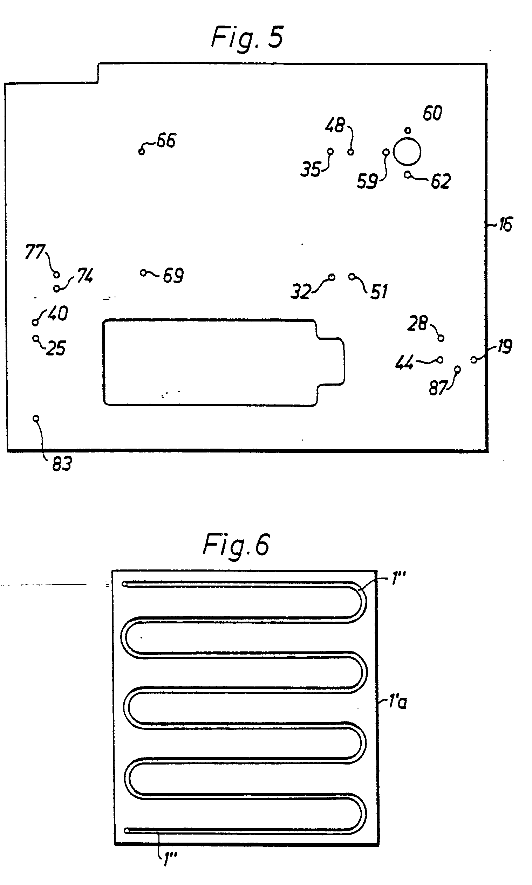

The dialyzer is preferably a plate dialyzer and built up in essentially the same way as the channel system. Consequently, the dialyzer comprises a rigid base plate, which may be part of the rigid top plate upon which the dialyzer is adapted to be arranged, and a hard top plate 1', and a soft intermediate plate 1'a clamped between these two plates and being provided with a channel 1" (Fig. 6) over which a semipermeable membrane is arranged in order to provide tight flow pathways on each side of the membrane for the complex medium and the dialysis solution, respectively.

For illustrative purpose, the channel system will be described with reference to Figs. 1—5 for the case where the device is adapted for measuring of the concentration of glucose in blood, whereby the following liquids are used, blood, dialysis solution, heparin and calibration solution.

1. *Blood Channel*

Blood is withdrawn from a patient by means of the cannula 10 and is passed into the channel system via the tube 12 and a not shown channel in the first connecting piece 14 in communication with an inlet opening 19 in the top plate. The inlet opening 19 is in communication with one end 20 of the groove 21 in the base plate 18 via a corresponding hole 22 in the intermediate plate 17. The other end 23 of the groove 21 is in communication with the left end of the tube 6 via a hole 24 in the intermediate plate, a corresponding hole 25 in the top plate and a corresponding not shown channel in the second connecting piece 15. The other end of the tube 6 is in communication with one end 26 of a groove 27 in the base plate via a not shown channel in the first connecting piece 14, a hole 28 in the top plate 16 and a corresponding hole 29 in the intermediate plate 17. The other end 30 of the groove 27 is in communication with an inlet to the blood side of the dialyzer 1, which preferably is of the kind as described above with reference to Fig. 6, through a hole 31 in the intermediate plate 17 and a corresponding hole 32 in the top plate 16. The outlet of the blood side of the dialyzer is in communication with one end 33 of the groove 34 in the base plate via a hole 35 in the top plate and a corresponding hole 36 in the intermediate plate, while the other end 37 of the groove 34 is in communication with a waste container (not shown) connectable to an outlet 38 in the second connecting piece 15 via a hole 39 in the intermediate plate 17, a corresponding hole 40 in the top plate 16 and a corresponding not shown channel in the second connecting piece 15.

### 2. Channel for Dialysis Solution

The channel for dialysis solution comprises an inlet 41 in the second connecting piece 15 in communication with the left end of the tube 5 through a corresponding not shown channel in the second connecting piece 15. The other end of the tube 5 is in communication with one end 42 of a groove 43 in the base plate 18 through a not shown channel in the first connecting piece 14, a corresponding hole 44 in the top plate and a corresponding hole 45 in the intermediate plate. The other end 46 of groove 43 is in communication with an inlet to the dialysis solution side of the dialyzer 1 through a hole 47 in the intermediate plate and a corresponding hole 48 in the top plate. The outlet from the dialysis solution side of the dialyzer is in communication with one end 49 of a groove 50 in the base plate through a corresponding hole 51 in the top plate and a corresponding hole 52 in the intermediate plate.

The other end 53 of the groove 50 can optionally be in communication with either one end 54 of a groove 55 in the base plate or one end 56 of a groove 57 in the base plate.

In the former case the communication is provided through a hole 58 in the intermediate plate 17 and a corresponding hole 59 in the top plate 16 and a corresponding not shown channel in the flow changing means 8 which through simple controlling can be set in a position such that the channel in the flow changing means is in communication with a hole 60 in the top plate and a corresponding hole 61 in the intermediate plate which in turn is in communication with said one end 54 of the groove 55.

In the second case the flow changing means 8 is instead set such that its channel, which, on the one hand, is in communication with the end 53 of groove 50, as explained above, and, on the other hand, will be in communication with the end 56 of the groove 57 through a hole 62 in the top plate and a corresponding hole 63 in the intermediate plate, which in turn is in communication with said one end 56 of the groove 57.

The other end 64 of the groove 55 is in communication with an inlet to the enzyme reactor 2 through a hole 65 in the intermediate plate 17 and a corresponding hole 66 in the top plate, while the outlet from the enzyme reactor 2 is in communication with one end 67 of the groove 68 in the top plate through a hole 69 in the top plate and a corresponding hole 70 in the intermediate plate.

As is shown in Fig. 3 groove 68 in the base plate is in communication with the groove 57 via a joint 71.

The other end 72 of groove 57 is in communication with an inlet to the measuring unit 3 through a hole 73 in the intermediate plate and a corresponding hole 74 in the top plate, while the outlet from the measuring unit is in communication with one end 75 of a groove 76 in the base plate through a hole 77 in the top plate and a corresponding 78 in the intermediate plate.

As is shown in Fig. 3 the groove 76 is in communication with the other end 37 of the described groove 34 for communication with said not shown waste container via the outlet 38.

### 3. Channel for Heparin

The channel for heparin comprises an inlet 79 in the second connecting piece 15 in communication with the left end of tube 4 through a corresponding not shown channel in the second connecting piece 15. The other end of tube 4 is in communication with a corresponding not shown channel in the first connecting piece, which channel in turn is in communication with tube 11 which in a known manner is in communication with tube 12 within the cannula 10. Thereby there is provided a mixing of heparin and blood which is adapted to be withdrawn from for example a patient by means of the cannula 10 and tube 12. The tube 12 is connectable to the inlet opening 19 via a not shown channel in the first connecting piece 14 through setting of the changing valve 13 as will be explained.

### 4. Channel for Calibration Solution

The channel for calibration solution comprises an inlet 80 in the second connecting piece 15 in communication with one end 81 of a groove 82 in the base plate through a hole 83 in the top plate and a corresponding hole 84 in the intermediate plate. The other end 85 of groove 82 is connectable to the inlet opening 19 in the top plate through a hole 86 in the intermediate plate, a corresponding hole 87 in the top plate and a corresponding not shown channel in the first connecting piece 14, whereby this channel can be connected to the corresponding not shown channel for the blood in the first connecting piece through controlling of the changing valve 13. Thereafter the channel for calibration solution follows the above described blood channel.

### Operation of the Device

The device according to the invention operates in the following manner when it is used for measuring of glucose in the blood of a patient.

### A. Calibration

During calibration the changing valve 13 is set in a position such that the communication between the tubes 11 and 12 and the channel system is interrupted. Calibration solution is pumped from a not shown container in communication with the inlet 80 in the second connecting piece 15 through the groove 82 into the first connecting piece 14, where the calibration solution is mixed with heparin which is simultaneously pumped from a not shown container in communication with the inlet 79 in the second connecting piece 15 through the tube 4 in communication with said not shown channel for calibration solution within the first connecting piece 14. From the connecting piece 14 the mixture is pumped through the groove 21, tube 6 and groove 27 into the dialyzer 1 and from the dialyzer via the groove 34 to the not shown waste container in communication with the outlet 38 in the second connecting piece 15. Simultaneously, dialysis solution is pumped from a not shown container in communication with the inlet 41 in the second connecting piece 15, through the tube 5 and via the first connecting piece 14 through the groove 43 into the dialyzer 1, where diffusion of glucose from the flowing mixture of heparin and calibration solution occurs via a semipermeable membrane to form a dialysate containing glucose. From the dialyzer 1 the formed dialysate is pumped through the groove 50 in contact with the temperature sensor 9 and through the deaerator 7, via the flow changing means 8 into the groove 55 and through the enzyme reactor 2 where conversion of glucose to a more easily measurable compound is provided. From the enzyme reactor 2 the dialysate is then pumped via the groove 68, 57 for contact with the measuring unit 3 where the concentration of said compound is measured as an indirect measure of the concentration of blood in the calibration solution. From the measuring unit 3 the dialysate is thereafter pumped through the groove 76 to the not shown waste container in communication with the outlet 38 in the second connecting piece 15.

In order to obtain a reference value (0-value) the flow changing means 8 is set in such a position that the dialysate from the groove 50 is instead pumped through the groove 57 past the enzyme reactor 2 directly to the measuring unit 3 where such a reference value is obtained. Thereafter the dialysate is pumped through the groove 76 to the not shown waste container via the outlet 38 in the second connecting piece 15.

B. *Measuring of Glucose in Blood*
During this measuring the changing valve 13 is set in a position such that the tubes 11 and 12 are in communication with the channel system, while the communication between the tube 5 for calibration solution and the channel system is interrupted. Heparin, which is pumped from the container into the tube 4 through the inlet 79 in the second connecting piece 15, is pumped through the first connecting 14 into the tube 11 which is in communication with the tube 12 in the cannula 10, where mixing with blood, which is withdrawn from the patient by means of the cannula 10, occurs. The mixture of blood and heparin is pumped from the cannula 10 through the tube 12 and through the first connecting piece 14 into the groove 21, through the tube 6 and into the groove 27 via the first connecting piece 14. From the groove 27 the mixture is pumped through the dialyzer 1, the groove 34 to the not shown waste container via the outlet 38 in the second connecting piece 15. Simultaneously, dialysis solution is pumped in exactly the same manner as described above in connection with the calibration, whereby diffusion glucose from the blood occurs within the dialyzer 1 via the semipermeable membrane to form a dialysate containing glucose. Again, the dialysate is alternately pumped from the dialyzer 1 through the groove 50 either to the groove 55 in the enzyme reactor 2, where conversion of glucose to a more easily measurable compound is provided, or from the enzyme reactor 2 via the groove 68, 57 to the measuring unit 3, where the concentration of said compound is measured as an indirect measure of the concentration of glucose in the blood. From the measuring unit 3 the dialysate is thereafter pumped to the not shown waste container via the outlet 38 in the second connecting piece 15. Alternatively, the dialysate is pumped from the groove 50 directly to the groove 57 via the flow changing means 8 past the enzyme reactor 2 to the measuring unit 3 via the groove 57 to obtain a reference value. From the measuring unit 3 the dialysate is then pumped via the groove 76 to the not shown waste container in communication with the outlet 38 in the second connecting piece 15.

Example of suitable container comprising a chamber for as well dialysis solution, heparin, calibration solution as waste may the container as described in SE—A—8104146. Preferably, this container is provided with level marking as described in SE—A—8104147.

Industrial Applicability
The device according to the invention is adapted for measuring of the concentration of a low molecular weight compound in a complex medium. More specifically, it is adapted for measuring of the concentration of blood of patient, but it can also be used for quantitative and/or qualitative determination of low molecular weight compounds in other complex media other than blood, for example microbiological culture chambers.

**Claims**

1. A device for measuring of concentration of a low molecular weight compound in a complex medium, comprising a dialyzer (1), an enzyme reactor (2) and a measuring unit (3) in communication with each other through a channel system, characterized in that said channel system is composed of grooves (21, 27, 34, 43, 50, 57, 68, 76, 82) which are arranged on one side of a base plate (18) and which are covered by one or more plates (16, 17) stacked over each other.

2. A device according to claim 1, characterized in that said base plate (18) is manufactured of rigid material, while the next covering plate (17) is manufactured of soft material.

3. A device according to claim 2, characterized in that the soft plate (17) is arranged between said base plate (18) and a top plate (16) of rigid material.

4. A device according to claim 3, characterized in that said dialyzer (1), enzyme reactor (2) and measuring unit (3) are arranged on the upper side of the top plate (16) and in communication with the respective groove in said base plate (18) via perforating holes in the intermediate soft plate (17) and the top plate (16).

5. A device according to any of the preceding claims, characterized in that the stack of plates (16, 17, 18) is clamped under pressure by means of a rivet joint, screw joint or welding joint.

6. A device according to any of claims 2—5, characterized in that the intermediate soft plate (17) extends in the form of perforated taps with close fitting into the respective holes of the top plate (16).

7. A device according to any of the preceding claims, characterized in that it comprises a temperature sensor (9) arranged between the top plate (16) and the intermediate soft plate (17) in contact with the dialysate, which is adapted to flow from the dialyzer (1).

8. A device according to any of the preceding claims, characterized in that the channel system is interrupted through a pump segment or tubes (4, 5, 6) arranged over a pump hole (P) through said plates (16, 17, 18).

9. A device according to any of the preceding claims, characterized in that it comprises an deaerator (7) consisting of a hole through the intermediate soft plate (17) and the top plate (16), which is covered by a hydrophobic membrane.

10. A device according to any of the preceding claims, characterized in that said dialyzer (1) comprises a soft plate (1'a) provided with a channel (1″), and a semipermeable membrane covering said channel, whereby the soft plate and the membrane are clamped between a rigid base plate and a rigid top plate (1') while forming tight flow pathways on each side of the membrane for the complex medium and dialysis solution, respectively.

11. A device according to claim 10, characterized in that the rigid base plate is part of the top plate (16) upon which the dialyzer is adapted to be arranged.

12. A device according to any of the preceding claims, characterized in that the enzyme reactor (2) is releasably arranged on the top plate (16).

**Patentansprüche**

1. Vorrichtung zur Messung der Konzentration einer Verbindung niedrigen Molekulargewichts in einem komplexen Medium, mit einem Dialysator (1), einem Enzymreaktor (2) und einer Meßeinheit (3), die miteinander über ein Kanalsystem in Verbindung stehen, dadurch gekennzeichnet, daß das Kanalsystem aus Nuten (21, 27, 34, 43, 50, 57, 68, 76, 82) zusammengesetzt ist, die auf einer Seite einer Grundplatte (18) angeordnet sind und von einer oder mehreren Platten (16, 17), die übereinandergestapelt sind, abgedeckt sind.

2. Vorrichtung nach Anspruch 1, dadurch gekennzeichnet, daß die Grundplatte (18) aus starrem Material hergestellt ist, während die nächste Abdeckplatte (17) aus weichem Material hergestellt ist.

3. Vorrichtung nach Anspruch 2, dadurch gekennzeichnet, daß die weiche Platte (17) zwischen der Grundplatte (18) und der Oberplatte (16) aus starrem Material angeordnet ist.

4. Vorrichtung nach Anspruch 3, dadurch gekennzeichnet, daß die Dialysator (1), der Enzymreaktor (2) und die Meßeinheit (3) auf der Oberseite der Oberplatte (16) angeordnet sind und sich mit der entsprechenden Nut in der Grundplatte (18) über Perforierlöcher in der weichen Zwischenplatte (17) und der Oberplatte (16) in Verbindung befinden.

5. Vorrichtung nach einem der vorhergehenden Ansprüche, dadurch gekennzeichnet, daß der Stapel von Platten (16, 17, 18) mittels einer Nietenverbindung, Schraubenverbindung oder Schweißverbindung unter Druck eingespannt ist.

6. Vorrichtung nach einem der Ansprüche 2 bis 5, dadurch gekennzeichnet, daß sich die Zwischenplatte (17) in Form perforierter Abgriffe bzw. Hähne mit dichten Anschlußstücken in die betreffenden Löcher der Oberplatte (16) erstreckt.

7. Vorrichtung nach einem der vorhergehenden Ansprüche, dadurch gekennzeichnet, daß sie einen Temperatursensor (9) aufweist, der zwischen der Oberplatte (16) und der weichen Zwischenplatte (17) in Berührung mit dem Dialysat angeordnet ist, welches aus dem Dialysator (1) fließen kann.

8. Vorrichtung nach einem der vorhergehenden Ansprüche, dadurch gekennzeichnet, daß das Kanalsystem über ein Pumpensegment oder Rohrleitungen (4, 5, 6) unterbrochen ist, welche über ein Pumpenloch (P) durch die Platten (16, 17, 18) hindurch angeordnet sind.

9. Vorrichtung nach einem der vorhergehenden Ansprüche, dadurch gekennzeichnet, daß sie eine Entlüftungseinrichtung (7) aufweist, die aus einem Loch durch die weiche Zwischenplatte (17) und die Oberplatte (16) besteht, welches von einer hydrophoben Membran abgedeckt ist.

10. Vorrichtung nach einem der vorhergehenden Ansprüche, dadurch gekennzeichnet, daß der Dialysator (1) eine weiche Platte (1'a) aufweist, die mit einem Kanal (1″) und einer den Kanal abdeckenden semipermeablen Membran versehen ist, wobei die weiche Platte und die Membran zwischen einer starren Grundplatte und einer starren oberplatte (1') eingeklemmt sind, während dichte Fließwege auf jeder Seite der Membran für das komplexe Medium bzw. die Dialyselösung gebildet sind.

11. Vorrichtung nach Anspruch 10, dadurch gekennzeichnet, daß die starre Basisplatte Teil der Oberplatte (16) ist, auf welcher der Dialysator angeordnet werden kann.

12. Vorrichtung nach einem der vorhergehenden Ansprüche, dadurch gekennzeichnet, daß der Enzymreaktor (2) lösbar auf der Oberplatte (16) angeordnet ist.

## Revendications

1. Dispositif de mesure de la concentration d'un composé de faible poids moléculaire dans un milieu complexe, comprenant un dialyseur (1), un réacteur à enzyme (2) et une unité de mesure (3) en communication les uns avec les autres par l'intermédiaire d'un système de canaux, caractérisé en ce que ce système de canaux est composé de gorges (21, 27, 34, 43, 50, 57, 68, 76, 82) qui sont disposées sur une face d'une plaque de base (18) et qui sont couvertes par une ou plusieurs plaques (16, 17) superposées les unes aux autres.

2. Dispositif suivant la revendication 1, caractérisé en ce que la plaque de base (18) est fabriquée en matière rigide, tandis que la plaque de recouvrement suivante (17) est fabriquée en matière souple.

3. Dispositif suivant la revendication 2, caractérisé en ce que la plaque souple (17) est placée entre la plaque de base (18) et une plaque supérieure (16) en maitière rigide.

4. Dispositif suivant la revendication 3, caractérisé en ce que le dialyseur (1), le réacteur à enzyme (2) et l'unité de mesure (3) sont disposés sur la face supérieure de la plaque supérieure (16) et en communication avec la gorge respective de la plaque de base (18) par l'intermédiaire de trous qui traversent la plaque intermédiaire souple (17) et la plaque supérieure (16).

5. Dispositif suivant l'une quelconque des revendications précédentes, caractérisé en ce que l'empilage de plaques (16, 17, 18) est serré sous pression au moyen d'une jonction rivetée, d'une jonction vissée ou d'une jonction soudée.

6. Dispositif suivant l'une quelconque des revendications 2 à 5, caractérisé en ce que la plaque intermédiaire souple (17) s'étend sous la forme de prises perforées à ajustement serré dans les trous respectifs de la plaque supérieure (16).

7. Dispositif suivant l'une quelconque des revendications précédentes, caractérisé en ce qu'il comprend un capteur de température (9) placé entre la plaque supérieure (16) et la plaque intermédiaire souple (17) en contact avec le dialysat, qui est prévu pour s'écouler à partir du dialyseur (1).

8. Dispositif suivant l'une quelconque des revendications précédentes, caractérisé en ce que le système de canaux est interrompu à travers un segment de pompe ou des tubes (4, 5, 6) disposés au-dessus d'un orifice de pompe (P), ménagé à travers les dites plaques (16, 17, 18).

9. Dispositif suivant l'une quelconque des revendications précédentes, caractérisé en ce qu'il comprend un désaréateur (7), constitué par un trou traversant la plaque intermédiaire souple (17) et la plaque supérieure (16) et qui est recouvert par une membrane hydrophobe.

10. Dispositif suivant l'une quelconque des revendications précédentes, caractérisé en ce que le dialyseur (1) comprend une plaque souple (1'a) comportant un canal (1″) et une membrane semi-perméable recouvrant ledit canal, de sorte que la plaque souple et la membrane sont serrées entre une plaque de base rigide et une plaque supérieure rigide (1') tout en définissant des passages de circulation étanches sur chaque face de la membrane, pour le milieu complexe et la solution de dialyse, respectivement.

11. Dispositif suivant la revendication 10, caractérisé en ce que la plaque de base rigide fait partie de la plaque supérieure (16) sur laquelle le dialyseur doit être placé.

12. Dispositif suivant l'une quelconque des revendications précédentes, caractérisé en ce que le réacteur à enzyme (2) est placé de façon démontable sur la plaque supérieure (16).

Fig.1

Fig. 2

0 102 458

Fig.3

Fig. 4

3

## Fig. 5

## Fig. 6